# EUROPEAN PATENT APPLICATION

(11) **EP 0 706 783 A1**
(43) Date of publication of application: **17.04.1996**
(21) Application number: 95202761.3
(22) Date of filing: 13.10.1995
(51) Int. Cl.: A61F 2/06, A61N 5/10

(54) **Stent which can be implanted into a body vessel**

(30) Priority: 13.10.1994 NL 9401690
(71) Applicant: CORDIS EUROPA N.V., NL-9301 LJ Roden (NL)
(72) Inventor: Glastra, Hendrik, NL-7535 PG Enschede (NL)
(74) Representative: 't Jong, Bastiaan Jacobus

(57) **Abstract**

The invention relates to a stent which can be implanted into a body vessel characterised in that it carries a detector for one or more body or environmental parameters. The stent comprises a storage or registration element for data provided by the detector as well as a transmission circuit for emitting electromagnetic radiation carrying information as regards the detected parameter.

## Description

The invention relates to a stent which can be implanted into a body vessel.

Many embodiments of such a stent are known, including those that are the subject-matter of EP-A-0 521 573 and EP-A-0 617 930, both in the name of the applicant.

Special possibilities will become available when according to the invention the active element comprises a detector for one or more body or environmental parameters as it will be possible to make observations or take measurements respectively, directly at a required location in the body of a patient.

According to the present invention the stent has been provided with a storage or registration element for data provided by the detector. Thus it will be possible to make observations over a certain period of time - during which the data are recorded - and to evaluate these after removal of the stent. Removal can for instance be effected in the manner described in the - not pre-published - Dutch patent application 9401571 in the name of the applicant.

The detector may be a radiation detector or a liquid flow detector, or may have been designed to detect the concentration of one of the blood components.

When the stent according to the invention comprises a radiation dosimeter it will be possible to record both the radiation emitted by an external source at the location of the stent and the radiation emitted by the stent itself when, as suggested by the invention, it has been provided with a source of radiation.

According to the invention the stent may have been provided with a transmission circuit for the purpose of emitting electromagnetic radiation, carrying information as regards the parameter detected. With the aid of modern microelectronics such a circuit can be made to have very small dimensions and the advantage of this embodiment is that from one moment to the next information as regards the detected parameters will be available.

The power supply of the required electronic circuits can be effected in the manner known from other applications by providing the stent with the combination of an excitation coil and a rectifier, excited by an external source of radiation.

The invention will be explained in greater detail with reference to the attached drawings.

Figure 1 shows a longitudinal cross-section of a stent provided with a radiation dosimeter.

Figure 2 shows a longitudinal cross-section of a stent provided with a detector for the purpose of detecting a parameter of the body fluids flowing past.

Figure 3 illustrates a schematic diagram of the components used in the embodiment according to figure 2.

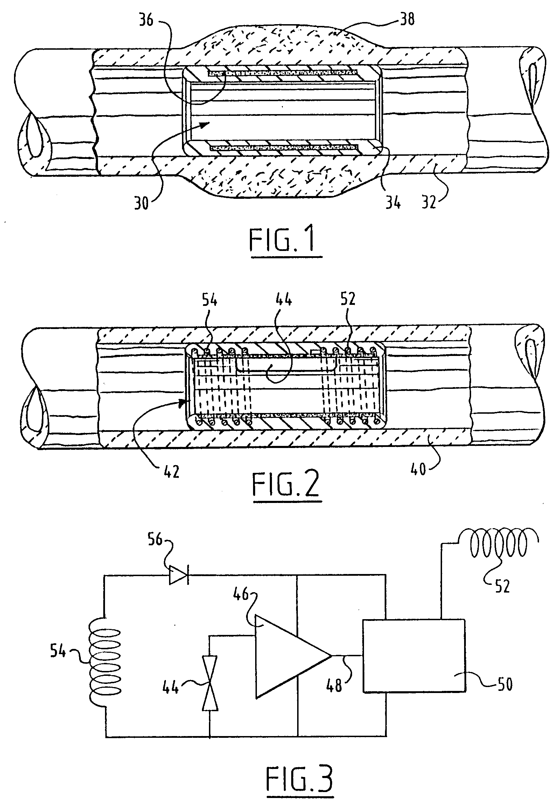

Figure 1 shows a longitudinal cross-section of a stent 30 received in a body vessel 32, in which a film-shaped radiation dosimeter 36 has been received in the body of the stent 34. Such a stent may for instance be placed inside a body vessel at the site of a tumour 38 surrounding this vessel and to be irradiated by means of an external source and will make it possible, on removal of the stent, to accurately establish the radiation dose to which the tumour has been exposed.

It is also possible to use the stent according to the invention in order to determine a parameter of the body fluids flowing past. Figure 2 and 3 relate to a stent suitable for such a purpose, of which a longitudinal cross-section is illustrated in figure 2.

The stent 42 received in the body vessel 40 is provided with a detection element 44, for instance a measuring device for the purpose of measuring the flow rate of body fluids flowing past the stent, such as for instance blood. As can be seen in figure 3, this measuring device 44 is connected to a signal processing circuit 46 of which the signal at the exit 48 is a measure of the flow rate.

This signal is fed to a coding and transmission circuit 50 which excites a transmission coil 52 received inside the stent. The electromagnetic radiation emitted carries - by means of modulation for instance - information as regards the measuring signal of the detector 44.

The power supply of the device may consist of a miniature battery not shown here, or it is possible to use a excitation coil 54 arranged inside the stent which is excited from the outside by means of electromagnetic radiation so that an alternating voltage is generated in the coil 54 which is rectified by means of a diode 56.

It may be clear that in addition to the examples described above, a great many other embodiments and applications are possible.

## Claims

1. Stent which can be implanted into a body vessel characterised in that it carries a detector for one or more body or environmental parameters.

2. Stent as claimed in claim 1, characterised in that it comprises a storage or registration element for data provided by the detector.

3. Stent as claimed in claim 1, characterised in that the detector is a radiation detector.

4. Stent as claimed in claim 1, characterised in that the detector is a liquid flow detector.

5. Stent as claimed in claim 1, characterised in that the detector has been designed to detect the concentration of one of the blood components.

6. Stent as claimed in claim 1, characterised in that it comprises a radiation dosimeter.

7. Stent as claimed in claim 1, characterised in that the stent comprises a transmission circuit for emitting electromagnetic radiation carrying information as regards the detected parameter.
